# EUROPÄISCHE PATENTSCHRIFT

(11) **EP 0 847 388 B1**
(45) Veröffentlichungstag und Bekanntmachung des Hinweises auf die Patenterteilung: **25.06.2003**
(21) Anmeldenummer: 96930102.7
(22) Anmeldetag: 26.08.1996
(51) Int. Cl.: C07D 213/82, A01N 43/40, C07D 277/56, A01N 43/78, C07D 231/14, A01N 43/56

(54) **HETEROZYKLISCH SUBSTITUIERTE BIPHENYLAMID DERIVATE, DEREN HERSTELLUNG UND DEREN VERWENDUNG ALS FUNGIZIDE**
HETEROCYCLICALLY SUBSTITUTED BIPHENYLAMIDE DERIVATIVES, THEIR PREPARATION AND THEIR USE AS FUNGICIDES
DERIVES DE BIPHENYLAMIDE A SUBSTITUTION HETEROCYCLIQUE, LEUR PREPARATION ET LEUR UTILISATION COMME FONGICIDES

(30) Priorität: 30.08.1995 DE 19531813
(43) Veröffentlichungstag der Anmeldung: 17.06.1998
(73) Patentinhaber: BASF AKTIENGESELLSCHAFT, 67056 Ludwigshafen (DE)
(72) Erfinder: EICKEN, Karl, D-67157 Wachenheim (DE); RANG, Harald, D-67122 Altrip (DE); HARREUS, Albrecht, D-67063 Ludwigshafen (DE); GÖTZ, Norbert, D-67547 Worms (DE); AMMERMANN, Eberhard, D-64646 Heppenheim (DE); LORENZ, Gisela, D-67434 Hambach (DE); STRATHMANN, Siegfried, D-67117 Limburgerhof (DE)
(86) Internationale Anmeldenummer: EP9603753
(87) Internationale Veröffentlichungsnummer: WO97008148

(56) Entgegenhaltungen:
- EP-A- 0 545 099
- EP-A- 0 589 301
- WO-A-95/25723
- DE-A- 2 417 216
- FR-A- 2 337 997
- CHEMICAL ABSTRACTS, vol. 123, no. 15, 9.Oktober 1995 Columbus, Ohio, US; abstract no. 198788n, YOSHIKAWA Y. ET AL.: "Preparation of thiazolecarboxamide derivatives as agrochemical fungicides" Seite 1246; Spalte 2; XP002019142 & JP,A,07 145 156 (MITSUI TOATSU CHEMICALS) 6.Juni 1995

## Beschreibung

Die vorliegende Erfindung betrifft Biphenylamide der allgemeinen Formel I 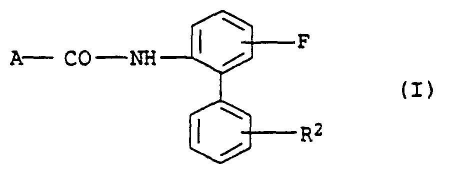 sowie deren Salze, in denen die Reste R¹, R² und A die folgenden Bedeutungen haben:
- R²: Wasserstoff, Halogen, C₁-C₄-Alkyl, Trifluormethyl, C₁-C₄-Alkoxy oder C₁-C₄-Alkylthio;
- A: 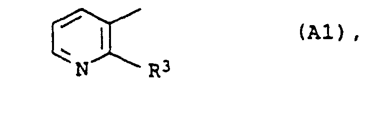 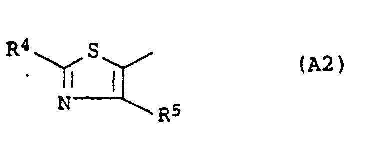 oder 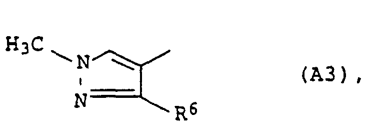
worin die Substituenten R³, R⁴, R⁵ und R⁶ ihrerseits bedeuten:
- R³: Chlor oder Trifluormethyl;
- R⁴: Wasserstoff oder Methyl;
- R⁵: Chlor, Methyl, Difluormethyl oder Trifluormethyl;
- R⁶: Methyl, Difluormethyl oder Trifluormethyl.

Außerdem betrifft die Erfindung ein Verfahren zur Herstellung der Verbindungen I, I enthaltende Mittel sowie ein Verfahren zur Bekämpfung von Schadpilzen und die Verwendung der Verbindungen I, ihrer Salze oder der Mittel hierzu.

Fungizide Biphenylamide des Typs I sind aus folgenden Druckschriften bekannt:

Die DE-A 24 17 216 beschreibt Pyridincarbonsäureamidderivate, die neben vielen anderen Substituenten auch durch einen Biphenyl-Rest an der Amidgruppe substituiert sein können.

Die FR-A-2 337 997 beschreibt Pyrazolcarbonsäureamidderivate, die neben einer Vielzahl anderer Substituenten auch durch einen Biphenyl-Rest an der Amidgruppe substituiert sein können.

Die EP-A-0 545 099 beschreibt Biphenylamide des Typs I, bei dem der Substituent R¹ Wasserstoff bedeutet.

Auch die EP-A-0 589 301 beschreibt Biphenylamide des Typs I, bei dem der Substituent R¹ Wasserstoff bedeutet.

Aus Chemical Abstracts, Vol. 123, no. 15, 09.10.1995, Abstract no. 198788n, Seite 1246 sind Biphenylamide des Typs I bekannt, bei dem die Substituenten R¹ und R² für Wasserstoff stehen.

Die in den zitierten Druckschriften genannten Wirkstoffe können jedoch hinsichtlich ihrer Wirkung noch nicht befriedigen.

Der vorliegenden Erfindung lagen daher Biphenylamide mit besserer Wirkung gegen Schadpilze als Aufgabe zugrunde.

Demgemäß wurde die eingangs definierten Verbindungen I gefunden.

Ferner wurden Mittel, welche die Verbindungen I oder deren Salze enthalten sowie ein Verfahren zur Herstellung von I und der Mittel gefunden. Des weiteren wurden ein Verfahren zur Bekämpfung von Schadpilzen sowie die Verwendung der Verbindungen I, ihrer Salze oder der Mittel hierzu gefunden.

Die Verbindungen I sind in an sich bekannter Weise aus den entsprechenden Carbonsäurehalogeniden II und den Biphenylaminen III unter Zuhilfenahme einer Base erhältlich. 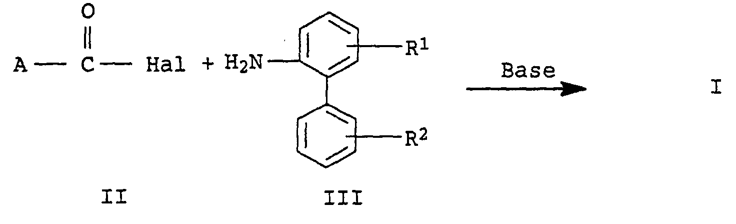 Hal Halogen, vorzugsweise Chlor oder Brom;
- R¹: Fluor;
- R²: Wasserstoff, Halogen, C₁-C₄-Alkyl, Trifluormethyl, C₁-C₄-Alkoxy oder C₁-C₄-Alkylthio;
- A: 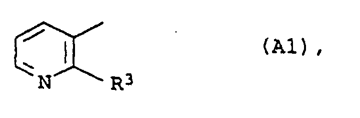 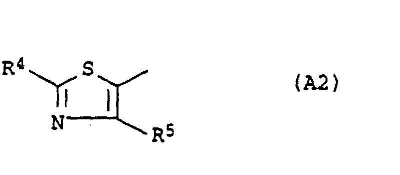 oder 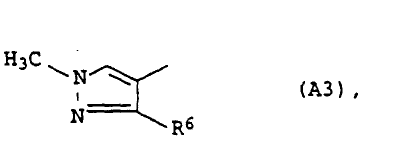
worin die Substituenten R³, R⁴, R⁵ und R⁶ ihrerseits bedeuten:
- R³: Chlor oder Trifluormethyl;
- R⁴: Wasserstoff oder Methyl;
- R⁵: Chlor, Methyl, Difluormethyl oder Trifluormethyl;
- R⁶: Methyl, Difluormethyl oder Trifluormethyl.

Hinsichtlich der Reaktionsbedingungen für die Herstellung der Verbindungen I und was die Herkunft der Ausgangsverbindungen II angeht vgl. z.B. EP-A 589 301 und EP-A 545 099.

Die Biphenylamine III sind allgemein bekannt oder in an sich bekannter Weise erhältlich (vgl. z.B. Tetrahedron Letters 28, Seite 5093 bis Seite 5096, 1987).

Teil der Erfindung sind auch die Salze der säurebeständigen Verbindungen I, welche basische Zentren, vor allem basische Stickstoffatome enthalten, insbesondere mit Mineralsäuren wie Schwefelsäure und Phosphorsäure oder Lewis-Säuren wie Zinkchlorid. Üblicherweise kommt es hierbei auf die Art des Salzes nicht an. Im Sinne der Erfindung sind solche Salze bevorzugt, die die von Schadpilzen freizuhaltenden Pflanzen, Flächen, Materialien oder Räume nicht schädigen und die Wirkung der Verbindungen I nicht beeinträchtigen. Besonders bedeutsam sind derartige, für landwirtschaftliche Zwecke geeignete Salze.

Die Salze der Verbindungen I sind in an sich bekannter Weise zugänglich, vor allem durch Umsetzen der entsprechenden Biphenylamide I mit den genannten Säuren in Wasser oder einem inerten organischen Lösungsmittel bei Temperaturen von -80 bis 120°C, vorzugsweise 0 bis 60°C.

Bei der eingangs angegebenen Definition der Verbindungen I wurden Sammelbegriffe verwendet, die repräsentativ für die folgenden Substituenten stehen:
Halogen: Fluor, Chlor, Brom und Jod;
Alkyl: geradkettige oder verzweigte Alkylgruppen mit 1 bis 8 Kohlenstoffatomen, z.B. C₁-C₆-Alkyl wie Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 2,2-Dimethylpropyl, 1,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Alkoxy: geradkettige oder verzweigte Alkoxygruppen mit 1 bis 4 Kohlenstoffatomen, z.B. C₁-C₃-Alkoxy wie Methyloxy, Ethyloxy, Propyloxy und 1-Methylethyloxy;
Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind, z.B. C₁-C₄-Alkylthio wie Methylthio, Ethylthio, Propylthio, 1-Methylethylthio, n-Butylthio und tert.-Butylthio.

Im Hinblick auf ihre biologische Wirkung gegen Schadpilze sind Verbindungen I bevorzugt, in denen R² steht für
- Halogen, vor allem Fluor, Chlor oder Brom;
- C₁-C₄-Alkyl, vor allem Methyl, Ethyl, Propyl, 1-Methylethyl, Butyl, 1-Methylpropyl, 2-Methylpropyl oder 1,1-Dimethylethyl;
- C₁-C₄-Alkoxy, vor allem Methoxy, Ethoxy, Propoxy, 1-Methylethoxy, Butoxy, 1-Methylpropoxy, 2-Methylpropoxy oder 1,1-Dimethylethoxy;
- Alkylthio, vor allem Methylthio, Ethylthio oder Propylthio.

Ganz besonders bevorzugt sind im Hinblick auf ihre Verwendung zur Bekämpfung von Schadpilzen die in den folgenden Tabelle 1 und 3 zusammengestellten Verbindungen I.

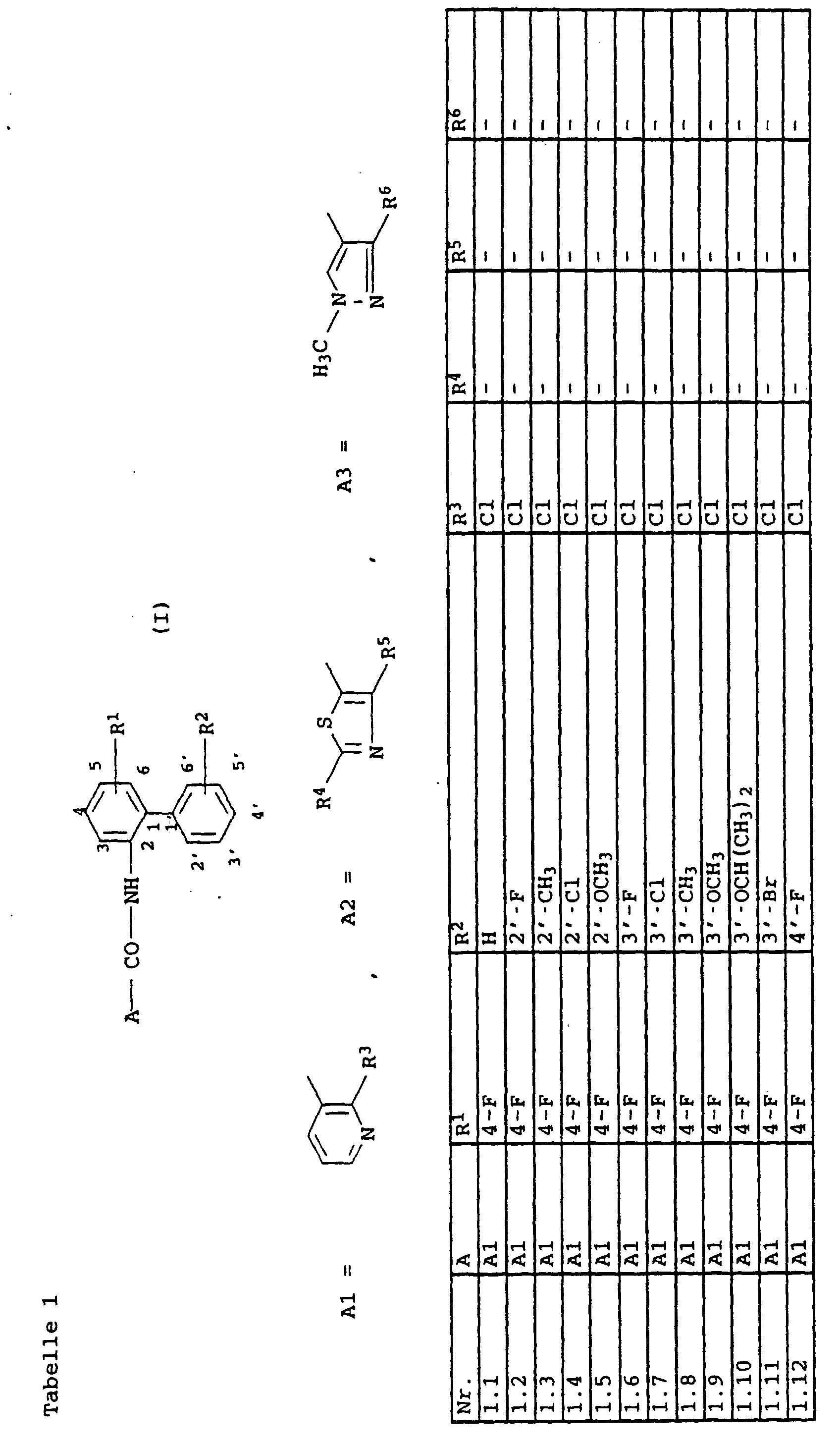

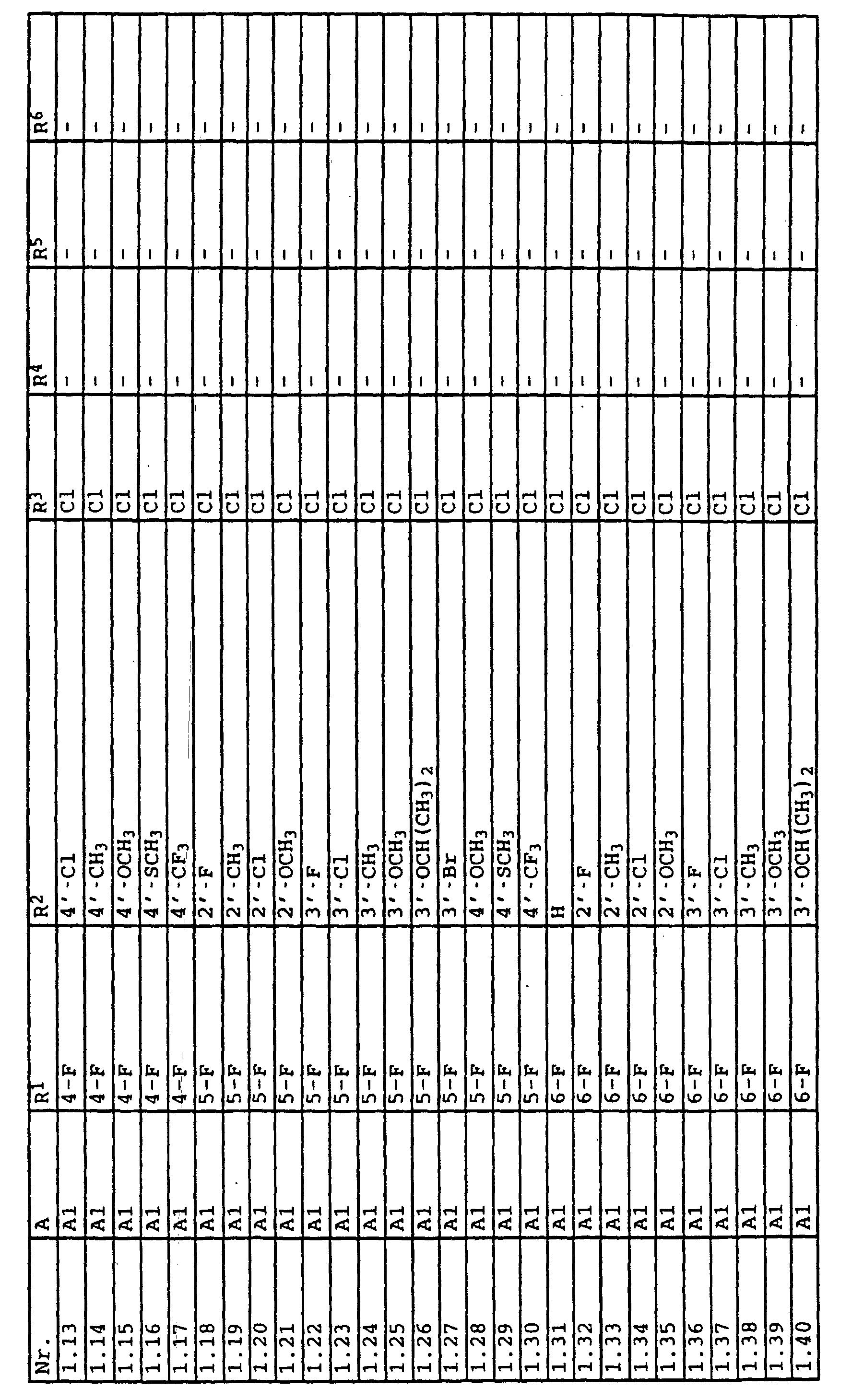

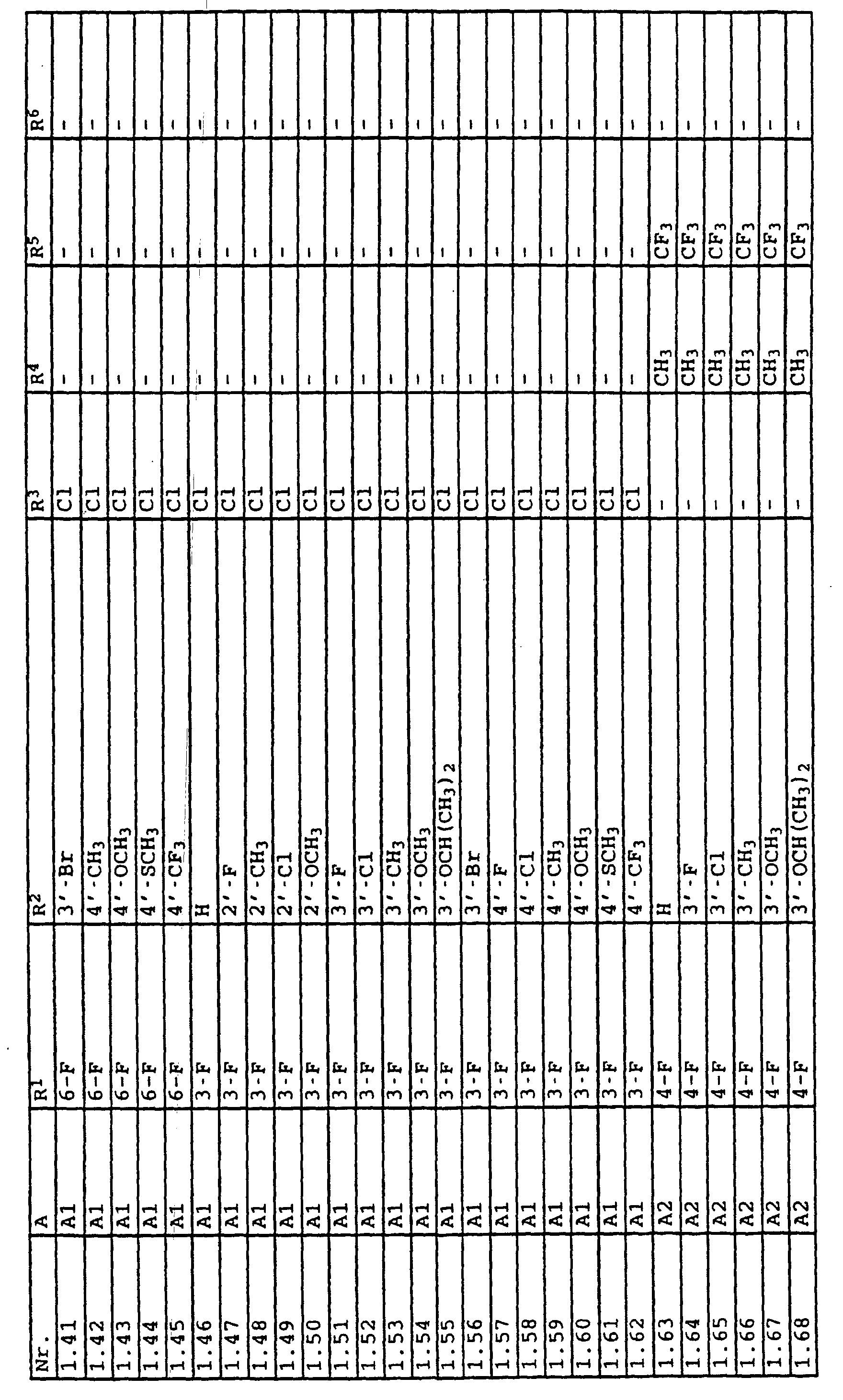

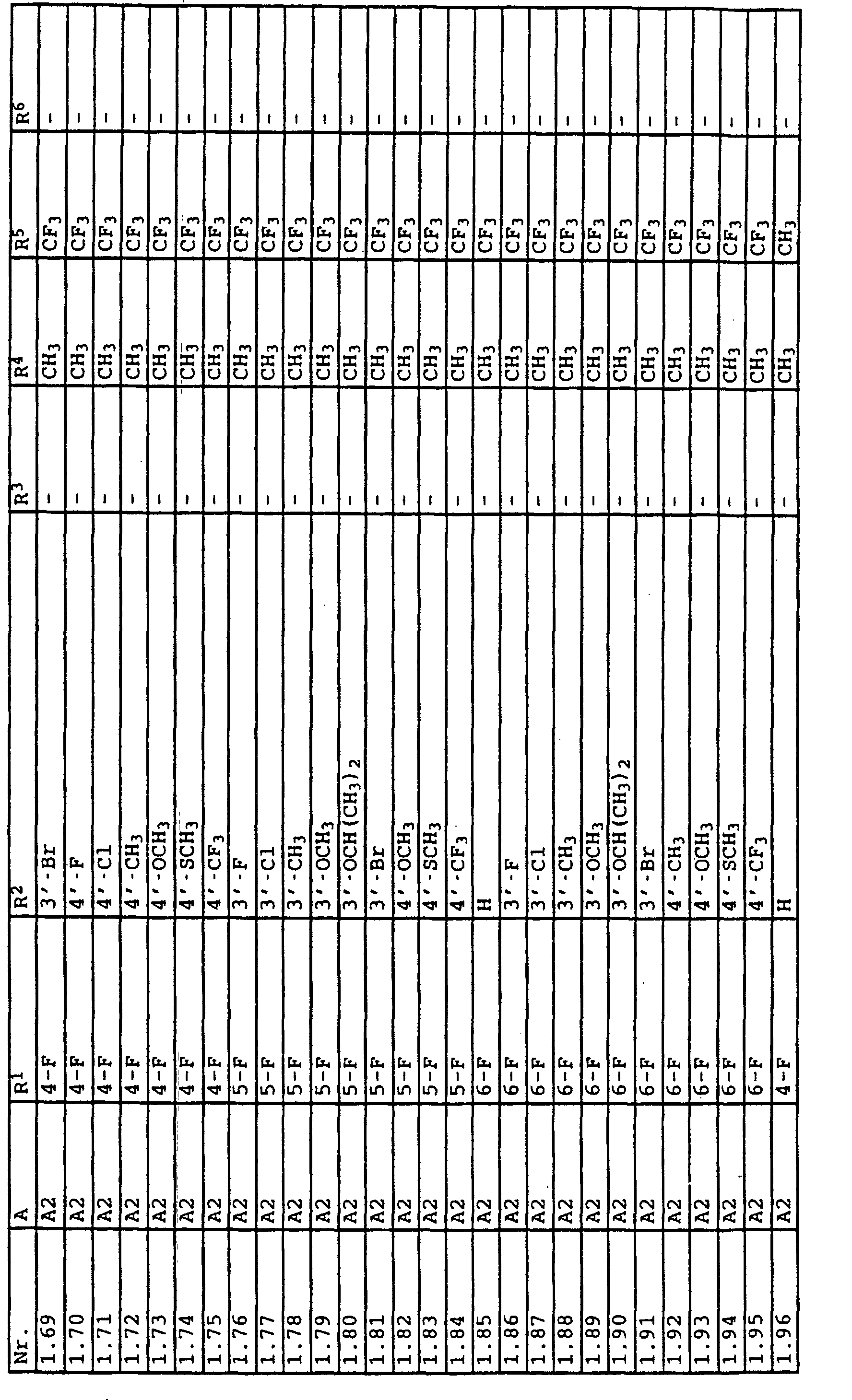

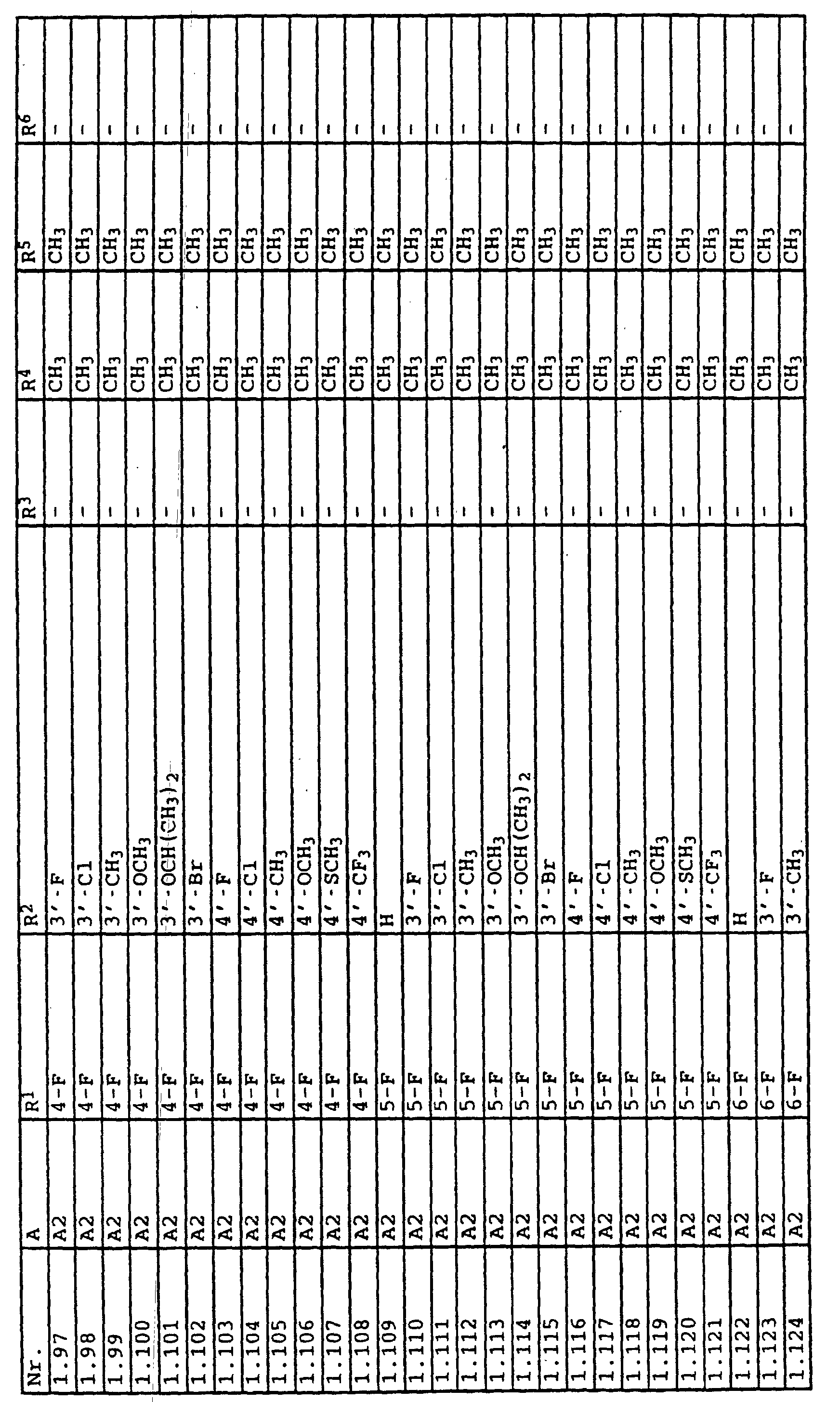

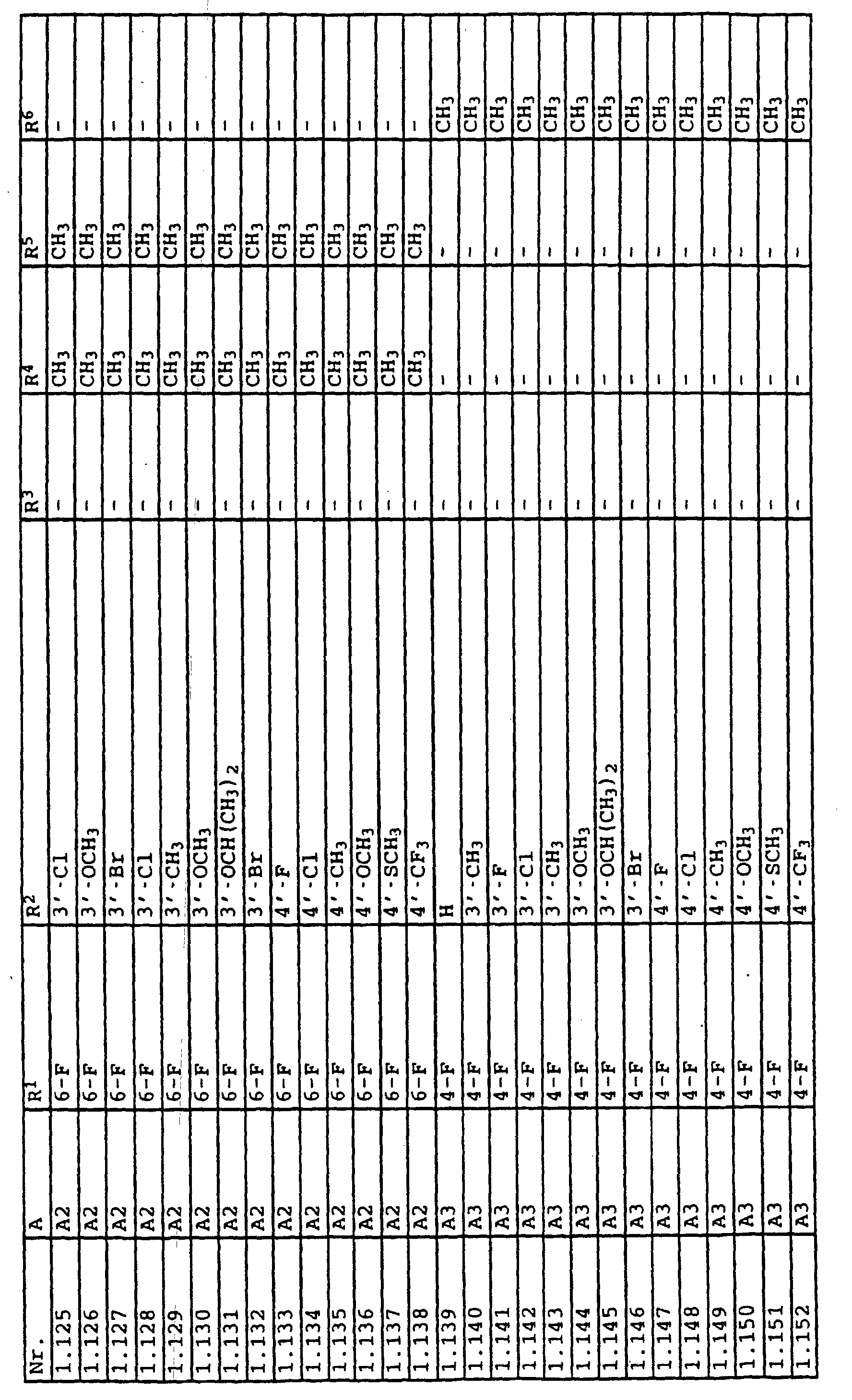

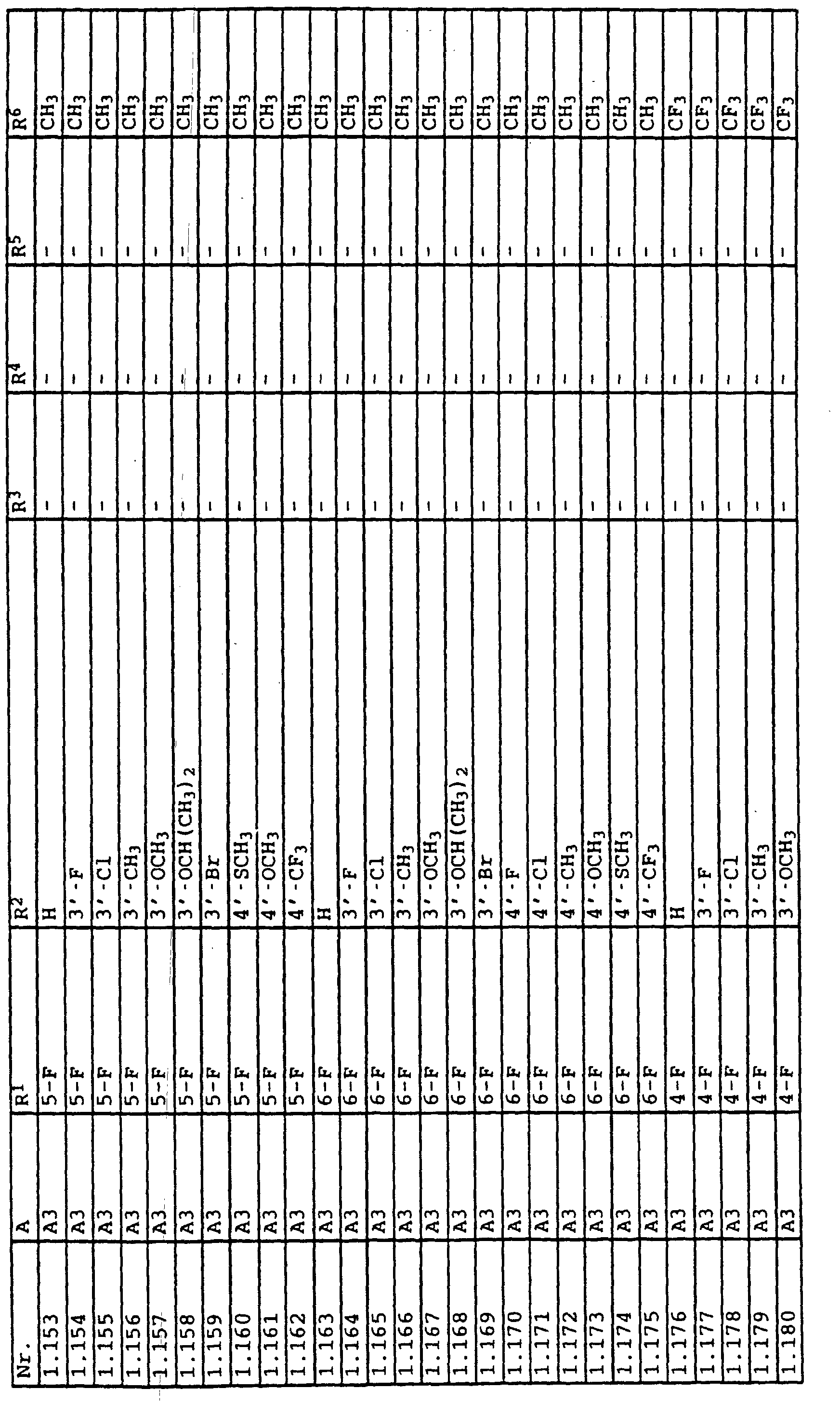

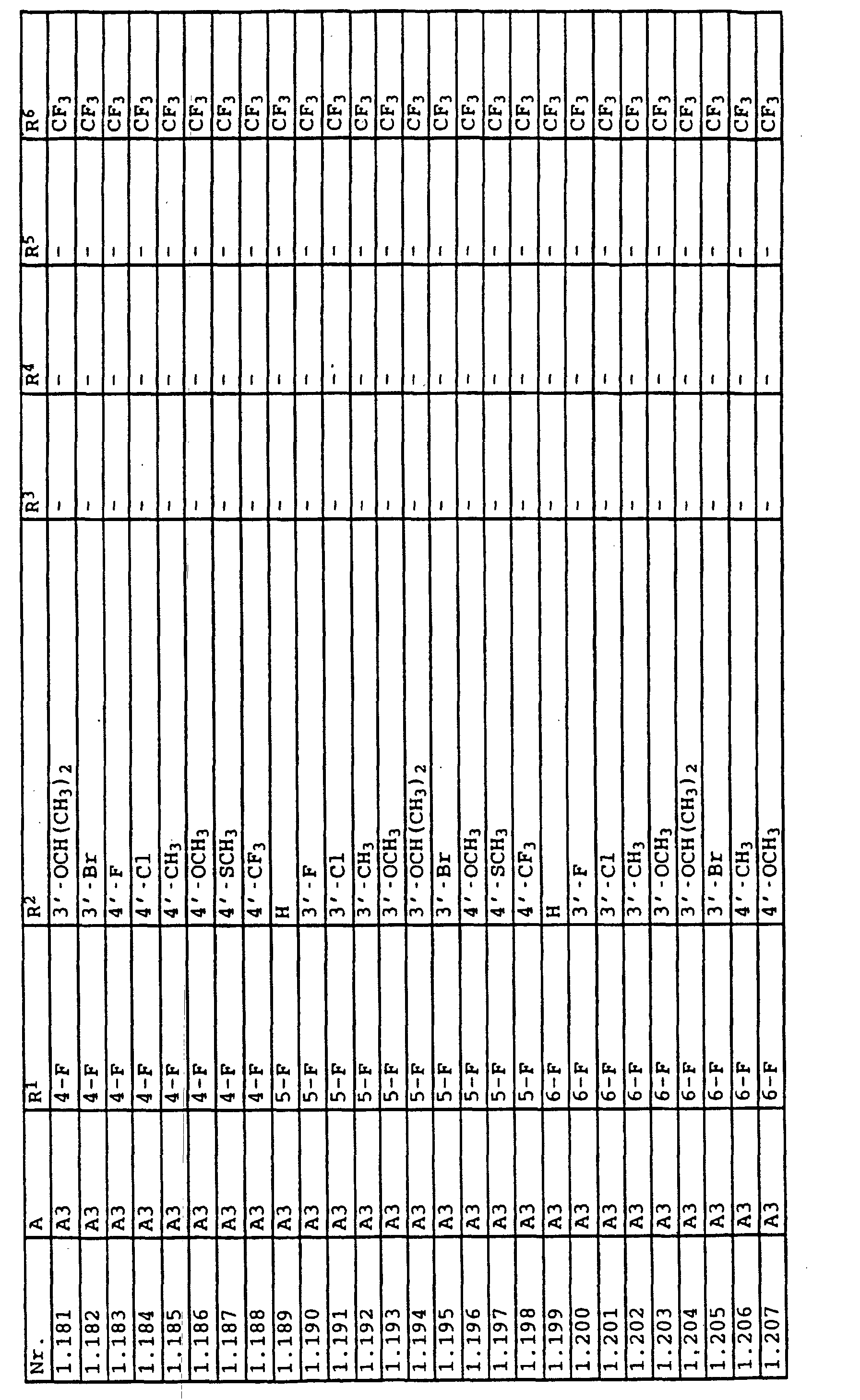

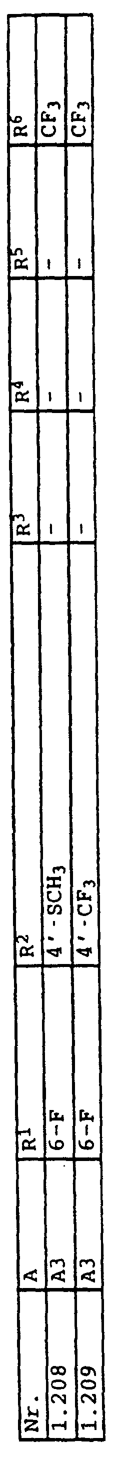

Die neuen Verbindungen der Formel I eignen sich als Fungizide.

Die neuen Verbindungen I und ihre Salze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden unter Verwendung üblicher Formulierungshilfsmittel - wie im folgenden beschrieben - und in an sich bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes iso-Octyl-, Octyl- oder Nonylphenol, Alkylphenol-, Tributylphenylpolyglykolether, Alkylarylpolyetheralkohole, iso-Tridecylalkohol, Fettalkoholethylenoxid-Kondensate, ethoxyliertes Rizinusöl, Polyoxyethylenalkyloder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
II. eine Mischung aus 10 Gew.-Teilen einer erfindungsgemäßen Verbindung I, 70 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 10 Gew.-Teilen einer erfindungsgemäßen Verbindung I, 40 Gew.-Teilen Cyclohexanon, 30 Gew.-Teilen iso-Butanol, 20 Gew.-Teilen des Anlagerungsproduktes von 40 mol Ethylenoxid an 1 mol Ricinusöl;
IV. eine wäßrige Dispersion aus 10 Gew.-Teilen einer erfindungsgemäßen Verbindung I, 25 Gew.-Teilen Cyclohexanol, 55 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen, vorzugsweise einer festen erfindungsgemäßen Verbindung I, 3 Gew.-Teilen des Natriumsalzes der Di-isobutylnaphthalin-2-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 62 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 50 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Deuteromyceten, Ascomyceten, Phycomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Schadpilze deren Lebensraum oder die von ihnen freizuhaltenden Pflanzen, Räume, Flächen oder Materialien mit einer wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:

Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Monilinia-Arten in Obst, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,025 und 2, vorzugsweise 0,1 bis 1 kg Wirkstoff pro ha.

Bei der Saatgutbehandlung werden im allgemeinen Wirkstoffmengen von 0,001 bis 50, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfid, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thion-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5, 6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethylfuran-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan,
2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-1H-1,2,4-triazol sowie
verschiedene Fungizide, wie Dodecylguanidinacetat, 3-[3-(3,5-Dimethyl-2-oxycyclohexyl)-2-hydroxyethyl]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[(3,5-Dichlorphenyl)-5-methyl-5-methoxymethyl-1,3-oxazolidin-2,4-dion, 3-(3,5-Dichlorphenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol,
Strobilurine wie Methyl-E-methoximino-[α-(o-tolyloxy)-o-tolyl)acetat, Methyl-E-2-{2-[6-(2-cyanophenoxy)pyridimin-4-yloxy]phenyl}-3-methoxyacrylat, Methyl-E-methoximino-[α-(2,5-dimethyloxy)-o-tolyl]acetamid.

Anilino-Pyrimidine wie N-(4,6-dimethylpyrimidin-2-yl)anilin, N-[4-methyl-6-(1-propinyl)pyrimidin-2-yl]anilin, N-(4-methyl-6-cyclopropyl-pyrimidin-2-yl)anilin.

Phenylpyrrole wie 4-(2,2-difluor-1,3-benzodioxol-4-yl)pyrrol-3-carbonitril.

Zimtsäureamide wie 3-(4-chlorphenyl)-3-(3,4-dimethoxyphenyl)acrylsäuremorpholid.

### Synthesebeispiele

Die in den nachstehenden Synthesebeispielen wiedergegebenen Vorschriften zur Herstellung der Verbindungen I und III können unter Abwandlung der Ausgangsverbindungen zur Gewinnung weiterer Vertreter der allgemeinen Formeln I oder III benutzt werden. Die physikalischen Daten der demgemäß hergestellten Produkte sind in den anschließenden Tabellen 2 und 3 z.T. mit angegeben.

### Beispiel 1. (Vorprodukt vom Typ III)

### 2-Amino-5,4'-difluorbiphenyl (Nr. 2.3 in Tabelle 2)

Zu einer Lösung von 11,4 g (0,060 mol) 2-Brom-4-fluoranilin in 120 ml 1,2-Dimethoxyethan gab man unter Stickstoff 2,4 g Tetrakis-(triphenylphosphin)-palladium 15,1 g (0,108 mol) 4-Fluorphenylboronsäure und eine Lösung von 30 g (0,282 mol) Natriumcarbonat in 120 ml Wasser und erhitzte 8 Stunden am Rückfluß. Nach dem Abkühlen wurden 200 ml Methyl-tert.-butylether und 100 ml Wasser zugegeben. Die organische Phase wurde zweimal mit je 120 ml wasser gewaschen, getrocknet und eingeengt. Nach Chromatographie des Rückstands an 50 g Kieselgel mit Cyclohexan als Laufmittel erhielt man 12,4 g der Titelverbindung (Fp: 67-69°C).

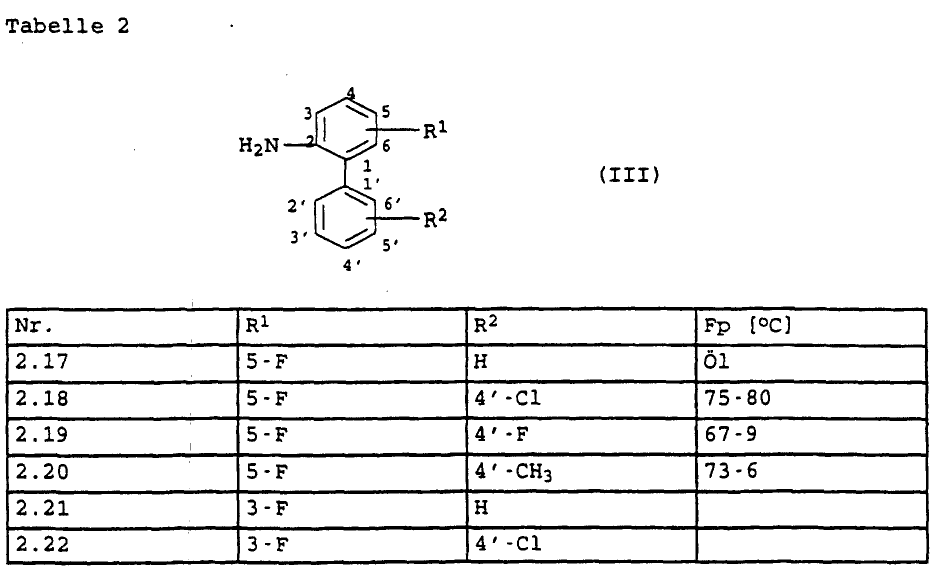

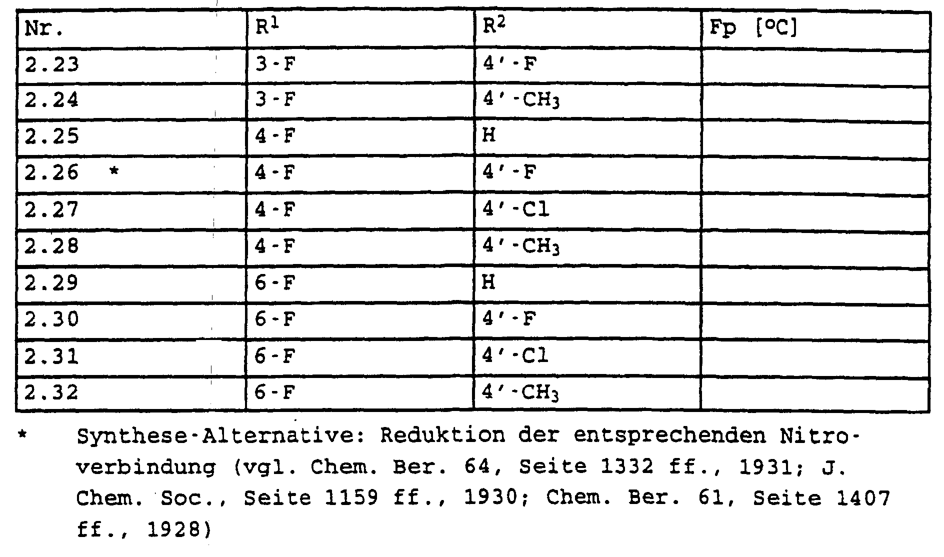

### Beispiel 2 (Wirkstoff vom Typ I)

### 2-Chlornicotinsäure-(4'-chlor-5-fluorbiphenyl)-2-amid

Zu einer Lösung von 1,55 g (7 mmol) 2-Amino-4'-chlor-5-fluor-biphenyl und 0,71 g (7 mmol) Triethylamin in 7 ml Tetrahydrofuran tropfte man bei + 5° C eine Lösung von 1,23 g (7 mmol) 2-Chlornicotinsäurechlorid in 3 ml Tetrahydrofuran und rührte 20 min bei + 5°C und 2 Stunden bei Raumtemperatur nach. Nach dem Einrühren in 140 ml Wasser wurde der Niederschlag abgesaugt. Durch Anteigen mit einer Mischung aus Diisopropylether und Cyclohexan (1 : 2) erhielt man 2,0 g der Titelverbindung (Fp: 131 - 136°C, Nr. 3.2 in Tabelle 3).

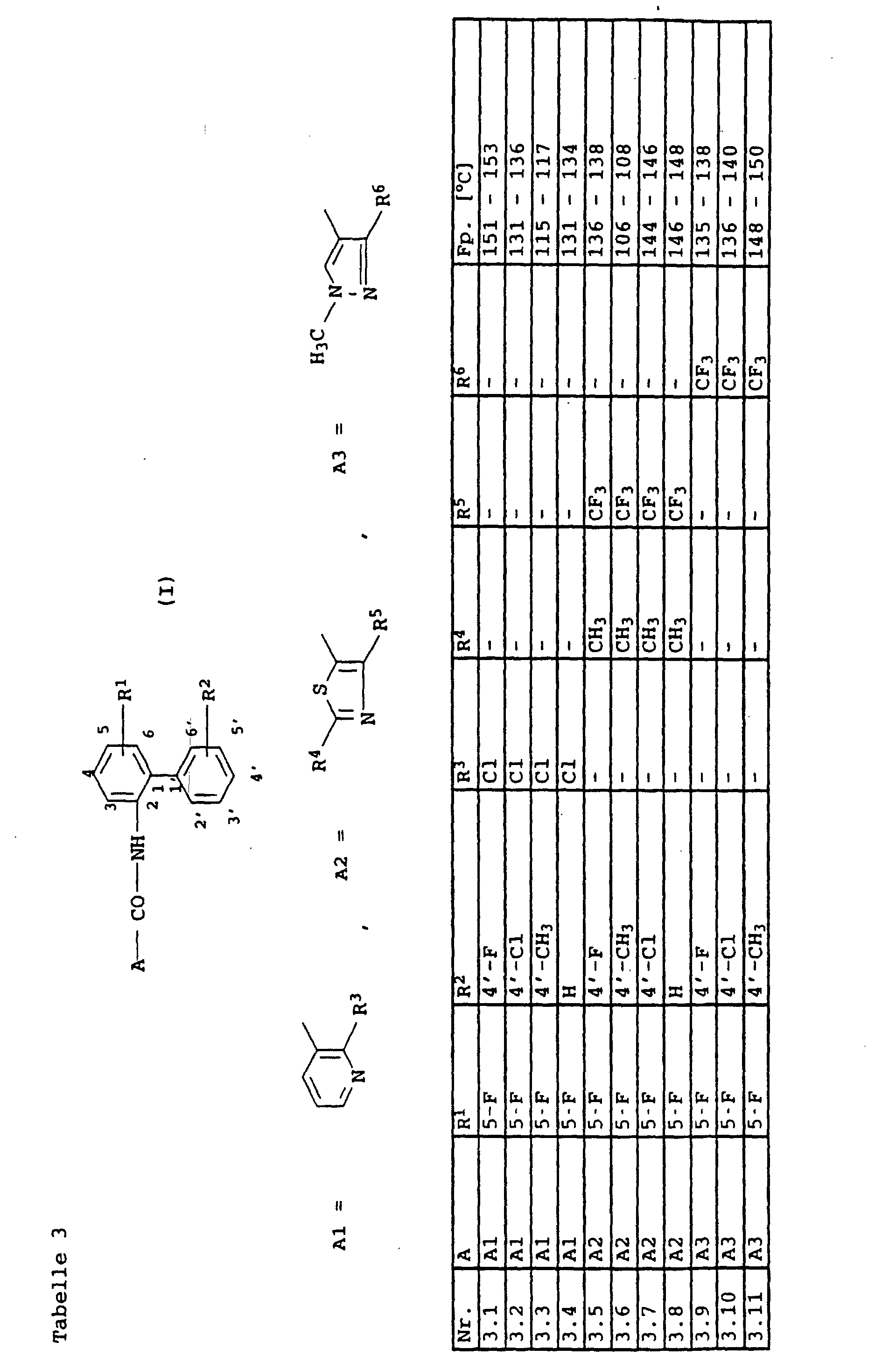

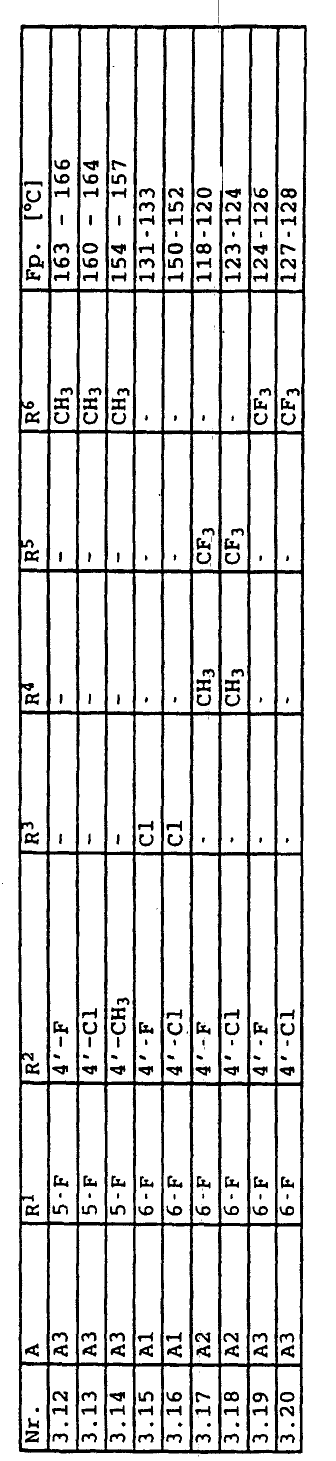

### Anwendungsbeispiele

Für die folgenden Versuche zur fungiziden Wirkung der Verbindungen I wurde eine Emulsion verwendet, welche zu 10 Gew.-% aus dem Wirkstoff und zu 90 Gew.-% aus einem Gemisch aus

| | |
|---|---|
| 70 Gew.-% | Cyclohexanol, |
| 20 Gew.-% | Nekanil® LN (Lutensol® AP6, Netzmittel mit Emulgier- und Dispergierwirkung auf der Basis ethoxylierter Alkylphenole) und |
| 10 Gew.-% | Emulphor® EL (Emulan® EL, Emulgator auf der Basis ethoxylierter Fettalkohole) |

bestand. Die gewünschten Wirkstoff-Konzentrationen wurden durch Verdünnen dieser Emulsion mit Wasser eingestellt.

Als Vergleichsverbindung "A" diente 2-Chlornicotinsäure-2'-ethylanilid, als Vergleichsverbindung "B" 2-Chlornicotinsäure-2'-phenylanilid. Beide Verbindungen sind bekannt aus der DE-A-24 17 216.

### Anwendungsbeispiel 1

### Botrytis cinerea

Scheiben von grünen Paprikaschoten wurden mit einer gemäß obiger Vorschrift hergestellten wäßrigen Aufbereitung tropfnaß gespritzt, welche jeweils 250 ppm eines einzigen Wirkstoffs enthielt. Als Wirkstoffe wurden folgende erfindungsgemäßen Verbindungen verwendet: 3.1, 3.2, 3.3, 3.4, 3.5, 3.6, 3.7, 3.9, 3.10, 3.11.

2 Stunden nach dem Antrocknen des Spritzbelags wurden die Fruchtscheiben mit einer Sporensuspension des Pilzes Botrytis cinerea, welche 1,7 x 10⁶ Sporen pro ml einer 2%-igen Biomalzlösung enthielt, inokuliert. Die Fruchtscheiben wurden anschließend 4 Tage bei 18°C in Kammern hoher Luftfeuchtigkeit inkubiert.

Die visuelle Bewertung ergab für die genannten Verbindungen einen Pilzbefall von 0-15% der Scheibenoberflächen.

Im Falle der Verbindung "A" lag der Pilzbefall unter ansonsten gleichen Versuchsbedingungen bei 100 %.

Scheiben, welche nicht mit einer Verbindung I oder der Verbindung "A" behandelt worden waren, wiesen einen Befall von 100 % auf.

### Anwendungsbeispiel 2

### Erysiphe graminis var. tritici

Blätter von in Töpfen gewachsenen Weizenkeimlingen (Sorte "Frühgold") wurden mit einer gemäß obiger Vorschrift hergestellten wäßrigen Aufbereitung besprüht, welche jeweils 250 ppm eines einzigen Wirkstoffs enthielt. Als Wirkstoffe wurden folgende erfindungsgemäßen Verbindungen verwendet: 3.2, 3.3, 3.5, 3.6, 3.7, 3.8, 3.9, 3.10, 3.11.

24 Stunden nach dem Antrocknen des Spritzbelags wurden die Blätter mit Oidien (Sporen) des Weizenmehltaus (Erysiphe graminis var. tritici) bestäubt. Die Pflanzen wurden anschließend für 7 Tage bei 20-22°C und einer relativen Luftfeuchtigkeit von 75-80% inkubiert.

Die visuelle Bewertung ergab für die genannten Verbindungen einen Pilzbefall von 5-25% der Blattoberfläche.

Im Falle der Verbindung "A" lag der Pilzbefall unter ansonsten gleichen Versuchsbedingungen bei 60 %. Für "B" wurde ein Befall von 80 % ermittelt.

Blätter welche nicht mit einer Verbindung I, "A" oder "B" behandelt worden waren, wiesen einen Befall von 80% auf.

## Patentansprüche

1. Biphenylamide der allgemeinen Formel I 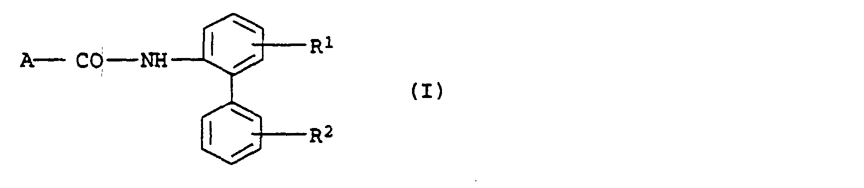 sowie deren Salze, in denen die Reste R¹, R² und A die folgenden Bedeutungen haben:
R¹ Fluor;
R² Wasserstoff, Halogen, C₁-C₄-Alkyl, C₁-C₄-Alkoxy oder C₁-C₄-Alkylthio;Trifluormethyl,
A 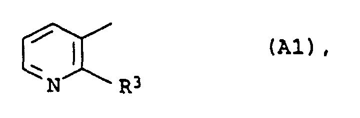 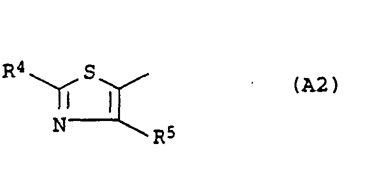 oder 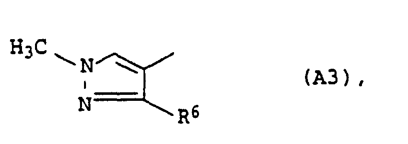
worin die Substituenten R³, R⁴, R⁵ und R⁶ ihrerseits bedeuten:
R³ Chlor oder Trifluormethyl;
R⁴ Wasserstoff oder Methyl;
R⁵ Chlor, Methyl, Difluormethyl oder Trifluormethyl;
R⁶ Methyl, Difluormethyl oder Trifluormethyl.

2. Verfahren zur Herstellung von Biphenylamiden der allgemeinen Formel I gemäß Anspruch 1, **dadurch gekennzeichnet, daß** man ein Carbonsäurehalogenid der allgemeinen Formel II 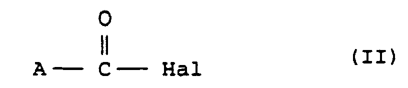 in der Hal für Halogen steht, mit einem Biphenylamin der allgemeinen Formel III 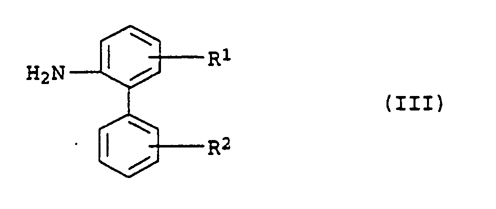 unter Zuhilfenahme einer Base umsetzt.

3. Zur Bekämpfung von Schadpilzen geeignete Mittel, enthaltend eine wirksame Menge mindestens einer Verbindung der allgemeinen Formel I oder eines ihrer Salze gemäß Anspruch 1 und mindestens ein übliches Formulierungshilfsmittel.

4. Verfahren zur Herstellung der Mittel gemäß Anspruch 3, **dadurch gekennzeichnet, daß** man eine fungizid wirksame Menge mindestens einer Verbindung der allgemeinen Formel I oder einem ihrer Salze gemäß Anspruch 1 mit mindestens einem üblichen Formulierungshilfsmittel in an sich bekannter Weise miteinander verarbeitet.

5. Verfahren zur Bekämpfung von Schadpilzen, **dadurch gekennzeichnet, daß** man die Schadpilze, deren Lebensraum oder die von ihnen freizuhaltenden Pflanzen, Räume, Flächen oder Materialien mit einer wirksamen Menge mindestens einer Verbindung der allgemeinen Formel I oder einem ihrer Salze gemäß Anspruch 1 oder mit einem I oder einem ihrer Salze enthaltenden Mittel gemäß Anspruch 3 behandelt.

6. Verwendung der Verbindungen der allgemeinen Formel I oder ihrer Salze gemäß Anspruch 1 oder der Mittel gemäß Anspruch 3 zur Bekämpfung von Schadpilzen.

## Claims

1. A biphenylamide of the general formula I 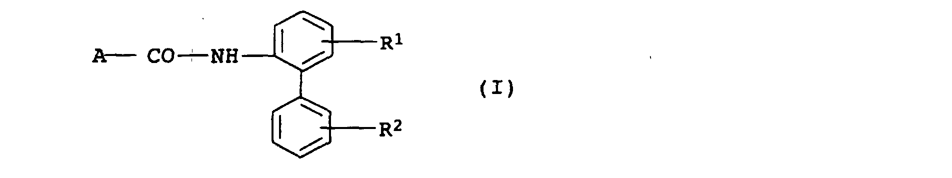 or a salt thereof where the radicals R¹, R² and A have the following meanings:
R¹ is fluorine;
R² is hydrogen, halogen, C₁-C₄-alkyl, trifluoromethyl, C₁-C₄-alkoxy or C₁-C₄-alkylthio;
A is 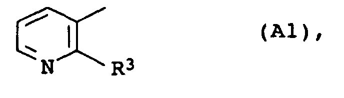 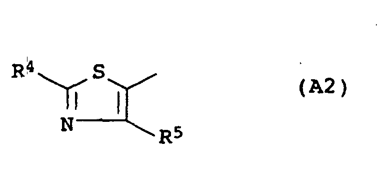 or 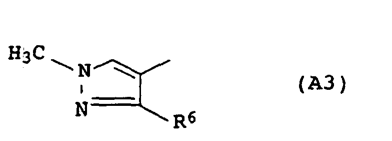
where the substituents R³, R⁴, R⁵ and R⁶, in turn, have the following meanings:
R³ is chlorine or trifluoromethyl;
R⁴ is hydrogen or methyl;
R⁵ is chlorine, methyl, difluoromethyl or trifluoromethyl;
R⁶ is methyl, difluoromethyl or trifluoromethyl.

2. A process for the preparation of a biphenylamide of the general formula I as claimed in claim 1, which comprises reacting a carboxylic acid halide of the general formula II 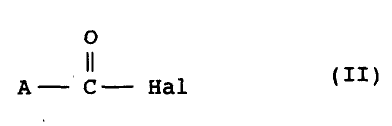 where Hal is halogen,
with a biphenylamine of the general formula III 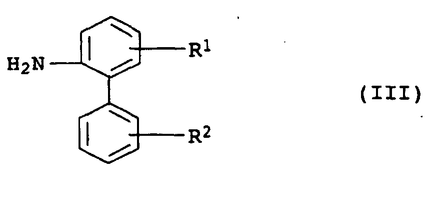 with the aid of a base.

3. A composition which is suitable for controlling harmful fungi, comprising an effective amount of at least one compound of the general formula I or of a salt thereof as claimed in claim 1 and at least one customary formulation auxiliary.

4. A process for the preparation of the composition as claimed in claim 3, which comprises processing a fungicidally active amount of at least one compound of the general formula I or of a salt thereof as claimed in claim 1 together with at least one customary formulation auxiliary in a manner known per se.

5. A method of controlling harmful fungi, which comprises treating the harmful fungi, their environment, or the plants, spaces, areas or materials to be kept free from them, with an effective amount of at least one compound of the general formula I or a salt thereof as claimed in claim 1 or with a composition comprising I or a salt thereof as claimed in claim 3.

6. The use of the compound of the general formula I or a salt thereof as claimed in claim 1 or of the composition as claimed in claim 3 for controlling harmful fungi.

## Revendications

1. Biphénylamides de formule générale I 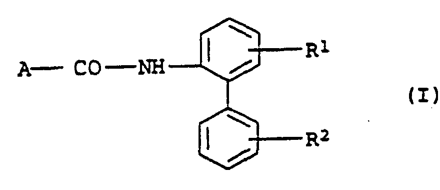 ainsi que leurs sels, dans lesquels les restés R¹, R² et A ont les significations suivantes:
R¹ fluoro;
R² hydrogène, halogéno, alkyle en C₁-C₄, trifluorométhyle, alcoxy en C₁-C₄, ou alkylthio en C₁-C₄;
A 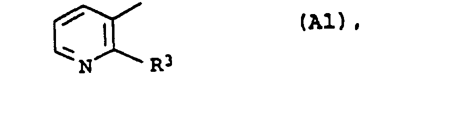 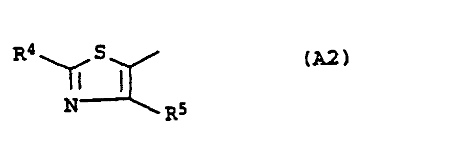 ou 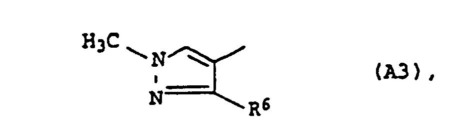
où les substituants R³, R⁴, R⁵ et R⁶ désignent à leur tour:
R³ chloro ou trifluorométhyle;
R⁴ hydrogène ou méthyle;
R⁵ chloro, méthyle, difluorométhyle ou trifluorométhyle;
R⁶ méthyle, difluorométhyle ou trifluorométhyle.

2. Procédé pour la préparation de biphénylamides de formule générale I selon la revendication 1, **caractérisé par le fait qu'**on fait réagir un halogénure d'acide carboxylique de formule générale II 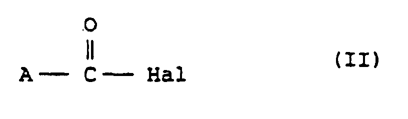 où Hal désigne un halogéno, avec une biphénylamine de formule générale III 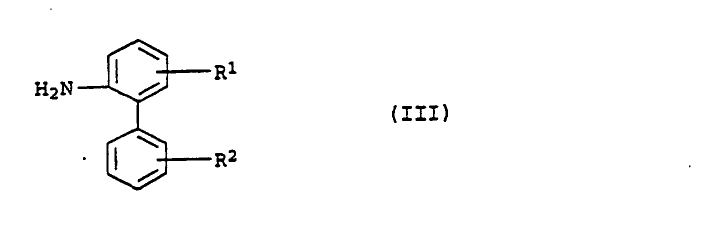 avec l'aide d'une base.

3. Produit approprié pour la lutte contre les champignons nuisibles, contenant une quantité efficace d'au moins un composé de formule générale I ou d'un de ses sels selon la revendication 1, et au moins un adjuvant de formulation classique.

4. Procédé pour la préparation des produits selon la revendication 3, **caractérisé par le fait qu'**on utilise ensemble, de manière connue en soi, une quantité efficace du point de vue fongicide d'au moins un composé de formule générale I ou d'un de ses sels selon la revendication 1 avec au moins un adjuvant de formulation classique.

5. Procédé pour la lutte contre les champignons nuisibles, **caractérisé par le fait qu'**on traite les champignons nuisibles, leur biotope ou les plantes, espaces, surfaces ou matériaux qui doivent ne pas en contenir avec une quantité efficace d'au moins un composé de formule générale I ou d'un de ses sels selon la revendication 1 ou avec un produit contenant I ou un de ses sels selon la revendication 3.

6. Utilisation des composés de formule générale I ou de leurs sels selon la revendication 1 ou des produits selon la revendication 3 pour la lutte contre les champignons nuisibles.
